# EUROPEAN PATENT APPLICATION

(11) **EP 4 792 731 A1**
(43) Date of publication of application: **19.08.2026**
(21) Application number: 24941301.4
(22) Date of filing: 05.08.2024
(51) Int. Cl.: A61B 1/303, A61B 1/06, A61B 1/32

(54) **WIRELESS ILLUMINATED VAGINAL SPECULUM UTILIZING MAGNETIC COUPLING RESONANCE PRINCIPLE**

(30) Priority: 27.05.2024 CN 202421163151 U
(71) Applicant: Gansu Maternal And Child Health Hospital (Gansu Provincial Central Hospital), Lanzhou, Gansu 730050 (CN)
(72) Inventor: DING, Zhongjun, Lanzhou, Gansu 730050 (CN); LIU, Peng, Lanzhou, Gansu 730050 (CN); LIU, Qing, Lanzhou, Gansu 730050 (CN); WANG, Zhiqiang, Lanzhou, Gansu 730050 (CN); YUE, Xiong, Lanzhou, Gansu 730050 (CN)
(74) Representative: Cleanthous, Marinos
(86) International application number: PCT/CN2024/109802
(87) International publication number: WO 2025/246023

(57) **Abstract**

A wireless illuminated vaginal speculum utilizing a magnetic coupling resonance principle is provided, including an upper blade and a lower blade. The lower blade is rotatably connected to the upper blade. A wireless receiving base is fixedly mounted at a position, close to a corner, of an upper part of the upper blade. A receiver coil is arranged inside the wireless receiving base. A lamp is fixedly mounted at a front end of the wireless receiving base. The receiver coil is electrically connected to the lamp. The vaginal speculum further includes a power supply base. A wireless transmitter is arranged inside the power supply base. A transmitter coil is arranged inside the wireless transmitter. The wireless transmitter and the wireless receiving base are provided.

## Description

### TECHNICAL FIELD

The present disclosure belongs to the technical field of medical instruments, and specifically, to a wireless illuminated vaginal speculum utilizing a magnetic coupling resonance principle.

### BACKGROUND

A speculum is widely used in an obstetrics and gynecology diagnosis and treatment process. The speculum needs to be used to assist in physical examinations and various gynecological surgical procedures.

Since a modern vaginal speculum came into being more than 200 years ago, the problem of illumination has consistently troubled clinicians. In current commonly used clinical treatment solutions, a light source is placed behind a surgeon or an operator. There are also some solutions for arranging lighting equipment on the speculum to address the above issue, but these solutions all require additional configuration of batteries or external wires to supply energy.

Most speculums currently used in clinical practice lack lighting devices, while speculums with lighting devices typically rely on battery power. To solve the lighting problem for surgical and operative fields of view, additional lighting equipment or batteries need to be configured. This type of speculum mainly has the following problems: 1. This type of lighting equipment is often heavy and difficult to move, making it fail to be used in special circumstances such as out-calls. 2. In an operation process, this type of lighting equipment is usually placed behind a doctor, making it hard for a light source to directly illuminate surgical and operative field-of-view regions. Furthermore, the body of an operator will inevitably block the light. As a result, the lighting effect is not ideal. 3. The mating costs are increased, and space resources in an operating room are also occupied. 4. Most speculums are disposable, so that using batteries to supply energy are cumbersome, many batteries will be used, and subsequent recycling treatment costs are high. 5. During use, most existing speculums are fixed by using rotary screw knobs, and angles between upper and lower blades are controlled. This wastes time and labor.

Therefore, there is an urgent clinical need for a disposable speculum that has its own light source without requiring a battery to supply energy or without wire connection to the outside.

In recent years, non-contact electric energy transmission technology has developed rapidly. Magnetic coupling resonant wireless electric energy transmission technology mainly uses a magnetic coupling resonance principle. Two electromagnetic systems with identical resonant frequencies and high quality factors are used. When a transmitter coil operates at a specific frequency, a receiver coil located at a distance away from the transmitter coil generates electromagnetic coupling resonance, and high-frequency electromagnetic energy undergoes large-scale exchange between the two coils. When the receiver coil is connected with a load, the load can absorb part of the energy, thus implementing wireless electric energy transmission. This wireless electric energy transmission technology eliminates the reliance on wires and batteries, and provides a brand-new solution for power supplying in special fields such as medical treatment. Its transmission distance can reach a meter level, while maintaining relatively high power and transmission efficiency. This technology is typically not affected by spatial obstacles. Consumed electric energy is only one millionth of that of traditional electromagnetic induction power supply technology. When a transmitting end is energized, the transmitter coil does not transmit electromagnetic waves to the outside, but instead generates a non-radiating magnetic field around. This magnetic field can be coupled with the receiver coil at the receiving end, to excite the receiver coil and supply electric energy to the circuit, thereby transmitting the electric energy with minimal costs. The intensity of the magnetic field is similar to the intensity of an earth magnetic field, so that the magnetic field will not cause adverse effects on the body and other equipment. At present, cardiac pacemakers and cochlear implant products have been powered by resonant magnetic coupling.

Therefore, applying the wireless electric energy transmission technology to the vaginal speculum can significantly solve the clinical usage problems faced by the traditional speculum.

### SUMMARY

The technical problem to be solved in the present disclosure is to provide a wireless illuminated vaginal speculum utilizing a magnetic coupling resonance principle for the shortcomings in the existing technology. A speculum that is in contact with a human body in the device is disposable, and a power supply base can be used for long time. The device is easy to operate and convenient to use, has good illumination effect, provides convenience for clinical operations, and can be popularized and applied.

To solve the above technical problems, a technical solution used in the present disclosure is as follows: A wireless illuminated vaginal speculum utilizing a magnetic coupling resonance principle includes an upper blade and a lower blade. Connecting holes are formed in two sides of the lower blade. Connecting pillars are fixedly mounted on two sides of the upper blade. The connecting pillars are rotatably mounted inside the connecting holes. The upper blade can rotate relative to the lower blade, so that a front end of the upper blade approaches or moves away from a front end of the lower blade. When the front end of the upper blade moves away, an effect of expansion is achieved, which facilitates use.

An observation window is formed in a position, close to a corner, of a middle part of the lower blade. When the front end of the upper blade moves away from the front end of the lower blade, observation or operation can be performed through the observation window.

A position, close to a corner, of an upper part of the upper blade is fixedly connected with a wireless receiving base. A receiver coil is arranged inside the wireless receiving base. A lamp is fixedly mounted at a front end of the wireless receiving base. The receiver coil is electrically connected to the lamp.

The wireless illuminated vaginal speculum further includes a power supply base. The power supply base includes a gel mat and a wireless transmitter. The wireless transmitter is embedded into the gel mat.

The wireless transmitter is provided with a transmitter coil. The transmitter coil is configured to convert electric energy into electromagnetic energy. The receiver coil is configured to convert electromagnetic energy into electric energy.

Resonant frequencies of a transmitting resonant circuit and a receiving resonant circuit that correspond to the transmitter coil and receiver coil are consistent. By using an electromagnetic coupling principle, an alternating magnetic field generated by a coil on a transmitting side of a power source is coupled to a coil on a receiving side of a non-contact load, thereby achieving non-contact electric energy transmission between the power source and the load. That is, coupled transmission of energy is achieved through same-frequency magnetic field resonance between the transmitter coil and the receiver coil. It features long transmission distance, high transmission power and high transmission efficiency, and is not affected by spatial obstacles.

The power supply base converts electric energy into the electromagnetic energy through the transmitter coil. The receiver coil generates induced current based on the electromagnetic induction principle, to provide the electric energy to the lamp. The lamp performs illumination from the upper blade, to facilitate surgical procedures while avoiding blocking of light by an external wire or other factors.

A mounting hole is formed in an upper side of a lower part of the lower blade. A strip-shaped hole is transversely formed in a lower part of the upper blade. An adjustment shaft is movably mounted inside the mounting hole and the strip-shaped hole. The adjustment shaft is fixedly provided with clamping plates. The clamping plates are symmetrically arranged on upper and lower sides of the adjustment shaft. Spacings between the clamping plates are the same. Clear distances between the clamping plates are equal to a wall thickness of the upper blade.

A height of the strip-shaped hole is equal to a diameter of the adjustment shaft, and a width of the strip-shaped hole is equal to edge distances between the clamping plates. The adjustment shaft is rotated. When the clamping plates are parallel to the strip-shaped hole, the clamping plates can exactly pass through the strip-shaped hole. In this case, the upper blade can rotate relative to the lower blade. With the rotation of the upper blade, the lower part of the upper blade is located between different clamping plates. In this case, the adjustment shaft is rotated such that the clamping plates are perpendicular to the strip-shaped hole, and the clamping plates clamp the upper blade. Thus, the upper blade cannot rotate relative to the lower blade. The clamping plates play a role in limiting and fixing the upper blade.

Preferably, a limiting block is fixedly mounted at one end of the adjustment shaft close to the lower blade, and a twist tab is fixedly mounted at another end of the transverse rod. The transverse rod can be rotated through the twist tab such that the clamping plates are parallel or perpendicular to the strip-shaped hole, thereby facilitating adjustment of position and angles of the upper blade and the lower blade and facilitating operations.

Preferably, a pressure sensor is arranged on the gel mat, and the pressure sensor is electrically connected to the power supply base through a control circuit. The gel mat is laid beneath the body of a patient. The power supply base is positioned to facilitate adjustment on the body position of the patient, so that the wireless transmitter can stably supply power to the wireless receiving base. When the pressure sensor has detected a pressure, the control circuit activates the power supply base, and the wireless transmitter inside the power supply base starts to work.

Preferably, the upper blade and lower blade are made of transparent medical-grade plastic, so that the costs are low, and it is convenient for observation.

Compared with the existing technology, the present disclosure has the following advantages:

The wireless transmitter and the wireless receiving base are provided. Through cooperation between the transmitter coil and the receiver coil, the power supply base can supply power to the lamp to directly perform illumination on the upper blade, thus avoiding a problem that illumination light is blocked. The illumination effect is good. The power supply base can be used for long time. The costs are low. The problems of a waste of resources and high costs due to use of a disposable battery are solved. The gel mat can be directly laid on a hospital bed. The gel mat is convenient to use, occupies a small space, has a small volume, is convenient for outdoor use, and is suitable for large-scale popularization.

The gel mat of the present disclosure is provided with the pressure sensor. During gynecological examinations or in a surgery, the gel mat bears the weight of the patient, the pressure sensor is pressed to activate a circuit, the wireless transmitter works, and the transmitter coil causes the receiver coil to generate electric energy through the electromagnetic coupling principle to turn on the lamp. After the operation is completed, the patient leaves the gel mat, the pressure sensor is no longer pressed, the wireless transmitter is powered off, and the lamp is turned off. The lamp is turned on or turned off automatically, so that electric energy is saved.

According to the present disclosure, the lower blade is provided with the adjustment shaft, and the adjustment shaft is provided with the clamping plates in an equal spacing manner. The upper blade can be clamped by the clamping plates, so that the upper blade cannot rotate and a positioning effect is achieved. The strip-shaped hole is formed in the upper blade, so that the adjustment shaft will not affect the rotation of the upper blade. During fixing of a distance between the upper blade and the lower blade, it is time-saving and labor-saving, and the vaginal speculum is easy to operate and convenient to use.

The present disclosure will be further explained in detail below in conjunction with the accompanying drawings and embodiments.

### BRIEF DESCRIPTION OF THE DRAWINGS

FIG. 1 is a schematic structural diagram of the present disclosure.
FIG. 2 is a schematic structural diagram of a lower blade in the present disclosure.
FIG. 3 is a schematic structural diagram of an upper blade in the present disclosure.

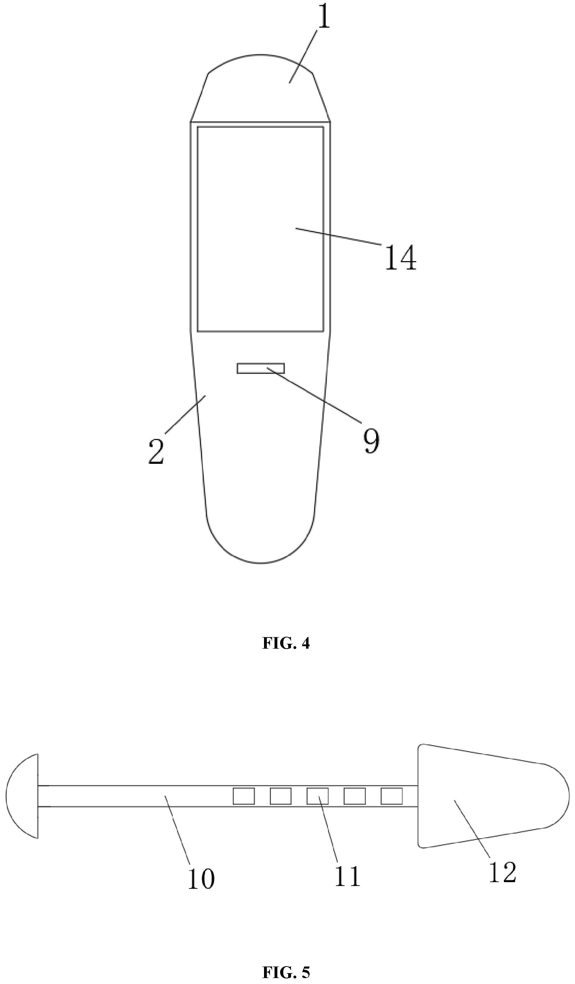

FIG. 4 is a front view of a lower blade in the present disclosure.
FIG. 5 is a schematic structural diagram of an adjustment shaft in the present disclosure.

### Reference numerals in the accompanying drawings:

| | | | | | |
|---|---|---|---|---|---|
| 1: | upper blade; | 2: | lower blade; | 3: | connecting pillar; |
| 4: | connecting hole; | 5: | wireless receiving base; | 6: | receiver coil; |
| 7: | lamp; | 8: | wireless transmitter; | 9: | strip-shaped hole; |
| 10: | adjustment shaft; | 11: | clamping plate; | 12: | twist tab; |
| 13: | gel mat; and | 14: | observation window. | | |

### DETAILED DESCRIPTION OF THE EMBODIMENTS

To make the objectives, features, and advantages of the present disclosure more comprehensible, specific implementations of the present disclosure are described in detail below in conjunction with the accompanying drawings. In the following description, many specific details are described for thorough understanding of the present disclosure. The present disclosure can, however, be embodied in many forms different from that described here. A person skilled in the art can make similar improvements without departing from the connotation of the present disclosure. Therefore, the present disclosure is not limited by the specific embodiments disclosed below.

It should be noted that when an element is referred to as being "fixed to" another element, the element can be directly on another component or there can be a centered element. When an element is considered to be "connected" to another element, the element can be directly connected to another element or there may be a centered element. The terms "perpendicular", "horizontal", "left", "right", and similar expressions used herein are for illustrative purposes only and do not necessarily represent the only implementation.

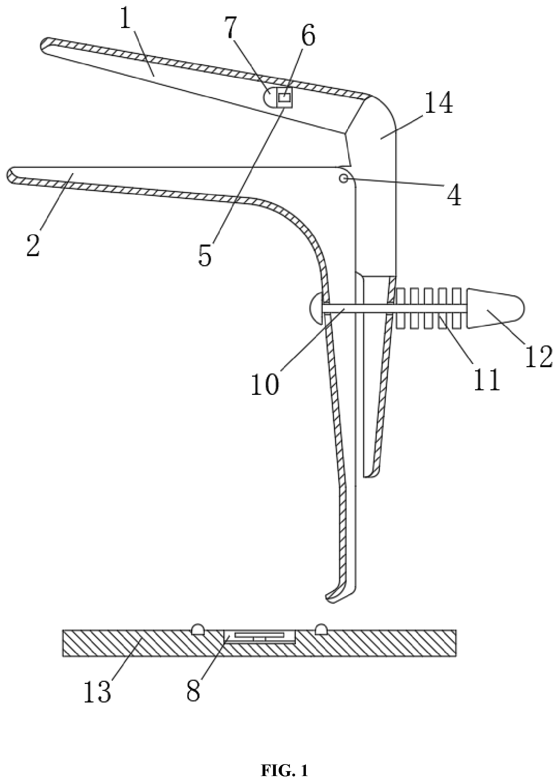

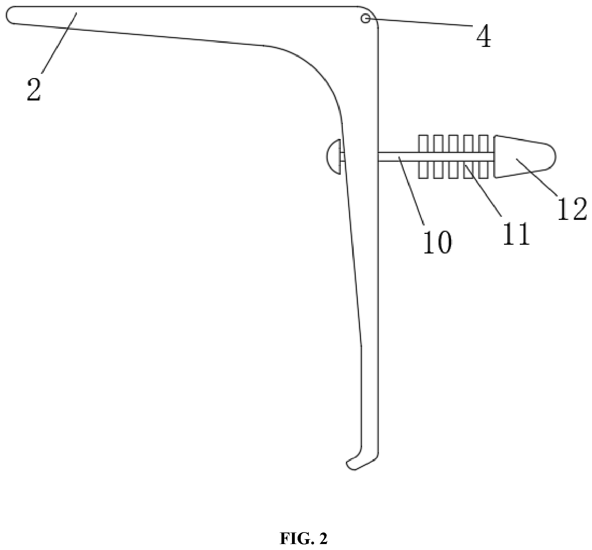

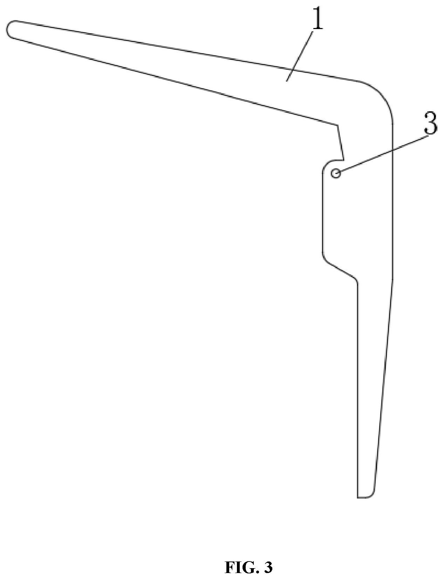

As shown in FIG. 1 to FIG. 3, the present disclosure provides a wireless illuminated vaginal speculum utilizing a magnetic coupling resonance principle includes an upper blade 1 and a lower blade 2. Connecting holes 4 are formed in two sides of the lower blade 2. Connecting pillars 3 are fixedly mounted on two sides of the upper blade 1. The connecting pillars 3 are rotatably mounted inside the connecting holes 4. The upper blade 1 can rotate relative to the lower blade 2, so that a front end of the upper blade 1 approaches or moves away from a front end of the lower blade 2. When the front end of the upper blade moves away, an effect of expansion is achieved, which facilitates use.

An observation window 14 is formed in a position, close to a corner, of a middle part of the lower blade 2. When the front end of the upper blade 1 moves away from the front end of the lower blade 2, observation or operation can be performed through the observation window 14.

A position, close to a corner, of an upper part of the upper blade 1 is fixedly connected with a wireless receiving base 5. A small-sized receiver coil 6 is arranged inside the wireless receiving base 5. A lamp 7 is fixedly mounted at a front end of the wireless receiving base 5. The receiver coil 6 is electrically connected to the lamp 7.

The wireless illuminated vaginal speculum further includes a power supply base. The power supply base includes a gel mat 13 and a wireless transmitter 8. The wireless transmitter 8 is embedded into the gel mat 13.

A transmitter coil is arranged inside the wireless transmitter 8. The transmitter coil is configured to convert electric energy into electromagnetic energy. The receiver coil 6 is configured to convert electromagnetic energy into electric energy.

Resonant frequencies of a transmitting resonant circuit and a receiving resonant circuit that correspond to the transmitter coil and receiver coil 6 are consistent, to obtain uniform inductance parameters and enhance energy transmission efficiency. By using an electromagnetic coupling principle, an alternating magnetic field generated by a coil on a transmitting side of a power source is coupled to a coil on a receiving side of a non-contact load, thereby achieving non-contact electric energy transmission between the power source and the load. That is, coupled transmission of energy is achieved through same-frequency magnetic field resonance between the transmitter coil and the receiver coil 6. It features long transmission distance, high transmission power and high transmission efficiency, and is generally not affected by spatial obstacles.

The power supply base converts electric energy into the electromagnetic energy through the transmitter coil. The receiver coil 6 generates induced current based on the electromagnetic induction principle, to provide the electric energy to the lamp 7. The lamp 7 performs illumination from the upper blade 1, to facilitate surgical procedures while avoiding blocking of light by an external wire or other factors.

A mounting hole is formed in an upper side of a lower part of the lower blade 2. A strip-shaped hole 9 is transversely formed in a lower part of the upper blade 1. An adjustment shaft 10 is movably mounted inside the mounting hole and the strip-shaped hole 9. The adjustment shaft 10 is fixedly provided with clamping plates 11. The clamping plates 11 are symmetrically arranged on upper and lower sides of the adjustment shaft 10. Spacings between the clamping plates 11 are the same. Clear distances between the clamping plates 11 are equal to a wall thickness of the upper blade 1.

A height of the strip-shaped hole 9 is equal to a diameter of the adjustment shaft 10, and a width of the strip-shaped hole 9 is equal to edge distances between the clamping plates 11. The adjustment shaft 10 is rotated. When the clamping plates 11 are parallel to the strip-shaped hole 9, the clamping plates 11 can exactly pass through the strip-shaped hole 9. In this case, the upper blade 1 can rotate relative to the lower blade 2. With the rotation of the upper blade 1, the lower part of the upper blade 1 is located between different clamping plates 11. In this case, the adjustment shaft 10 is rotated such that the clamping plates 11 are perpendicular to the strip-shaped hole 9, and the clamping plates 11 clamp the upper blade 1. Thus, the upper blade 1 cannot rotate relative to the lower blade 2. The clamping plates 11 play a role in limiting and fixing the upper blade 1.

In this embodiment, a limiting block is fixedly mounted at one end of the adjustment shaft 10 close to the lower blade 2, and a twist tab 12 is fixedly mounted at another end of the transverse rod 10. The transverse rod 10 can be rotated through the twist tab 12 such that the clamping plates 11 are parallel or perpendicular to the strip-shaped hole 9, thereby facilitating adjustment of position and angles of the upper blade 1 and the lower blade 2 and facilitating operations.

In this embodiment, a pressure sensor is arranged on the gel mat 13, and the pressure sensor is electrically connected to the power supply base through a control circuit. The gel mat 13 is laid beneath the body of a patient. The power supply base is positioned to facilitate adjustment on the body position of the patient, so that the wireless transmitter 8 can stably supply power to the wireless receiving base 5. When the pressure sensor has detected a pressure, the control circuit activates the power supply base, and the wireless transmitter 8 inside the power supply base starts to work.

In this embodiment, the upper blade 1 and lower blade 2 are made of transparent medical-grade plastic, so that the costs are low, and it is convenient for observation.

During use, the gel mat 13 is laid beneath the body of the patient, and a position of the power supply base is determined.

After the patient lies down on the gel mat 13, the pressure sensor has detected a pressure, the control circuit activates the power supply base, and the wireless transmitter 8 inside the power supply base starts to work. The transmitter coil inside the wireless transmitter 8 converts the electric energy into the electromagnetic energy.

First, the front end of the upper blade 1 and the front end of the lower blade 2 are in close fit with each other, and then the front end of the upper blade 1 and the front end of the lower blade 2 are placed into the vagina of the patient.

The adjustment shaft 10 is rotated. When the clamping plates 11 are parallel to the strip-shaped hole 9, the clamping plates 11 can exactly pass through the strip-shaped hole 9. In this case, the upper blade 1 can rotate relative to the lower blade 2.

When the vagina of the patient is expanded to an appropriate degree, the adjustment shaft 10 is rotated, so that the clamping plates 11 are perpendicular to the strip-shaped hole 9. In this case, the upper blade 1 cannot rotate relative to the lower blade 2, and the clamping plates 11 play a role in limiting and fixing the upper blade 1.

In this case, the wireless receiving base 5 is located above the power supply base. The receiver coil 6 inside the wireless receiving base 5 generates induced current based on the electromagnetic induction principle, to provide the electric energy to the lamp 7. The lamp 7 performs illumination from the upper blade 1, to facilitate surgical procedures while avoiding blocking of light by an external wire or other factors.

In this case, the front end of the upper blade 1 moves away from the front end of the lower blade 2, and observation or operation can be performed through the observation window 14.

After surgery ends, the patient leaves the gel mat 13, the pressure sensor has not detected a pressure, and the power supply base stops working.

The above descriptions are merely the preferred embodiments of the present disclosure and are not intended to make any limitation on the present disclosure. Any simple modifications, alterations, or equivalent changes made to the above embodiments according to the technical essence of the present disclosure shall all fall within the protection scope of the technical solutions of the present disclosure.

## Claims

1. A wireless illuminated vaginal speculum utilizing a magnetic coupling resonance principle, comprising an upper blade (1) and a lower blade (2), wherein connecting holes (4) are formed in two sides of the lower blade (2); connecting pillars (3) are fixedly mounted on two sides of the upper blade (1); the connecting pillars (3) are rotatably mounted inside the connecting holes (4); an observation window (14) is formed in a position, close to a corner, of a middle part of the lower blade (2);
a position, close to a corner, of an upper part of the upper blade (1) is fixedly connected with a wireless receiving base (5); a receiver coil (6) is arranged inside the wireless receiving base (5); a lamp (7) is fixedly mounted at a front end of the wireless receiving base (5); the receiver coil (6) is electrically connected to the lamp (7);
the wireless illuminated vaginal speculum further comprises a power supply base; the power supply base comprises a gel mat (13) and a wireless transmitter (8); the wireless transmitter (8) is embedded into the gel mat (13);
the wireless transmitter is provided with a transmitter coil; the transmitter coil is configured to convert electric energy into electromagnetic energy; and the receiver coil (6) is configured to convert electromagnetic energy into electric energy.

2. The wireless illuminated vaginal speculum utilizing the magnetic coupling resonance principle according to claim 1, wherein a mounting hole is formed in an upper side of a lower part of the lower blade (2); a strip-shaped hole (9) is transversely formed in a lower part of the upper blade (1); an adjustment shaft (10) is movably mounted inside the mounting hole and the strip-shaped hole (9); the adjustment shaft (10) is fixedly provided with clamping plates (11); the clamping plates (11) are symmetrically arranged on upper and lower sides of the adjustment shaft (10); spacings between the clamping plates (11) are the same; and clear distances between the clamping plates (11) are equal to a wall thickness of the upper blade (1).

3. The wireless illuminated vaginal speculum utilizing the magnetic coupling resonance principle according to claim 2, wherein a height of the strip-shaped hole (9) is equal to a diameter of the adjustment shaft (10), and a width of the strip-shaped hole (9) is equal to edge distances between the clamping plates (11).

4. The wireless illuminated vaginal speculum utilizing the magnetic coupling resonance principle according to claim 2, wherein a limiting block is fixedly mounted at one end of the adjustment shaft (10) close to the lower blade (2), and a twist tab (12) is fixedly mounted at another end of the adjustment shaft (10).

5. The wireless illuminated vaginal speculum utilizing the magnetic coupling resonance principle according to claim 1, wherein a pressure sensor is arranged on the gel mat (13), and the pressure sensor is electrically connected to the power supply base through a control circuit.

6. The wireless illuminated vaginal speculum utilizing the magnetic coupling resonance principle according to claim 1, wherein the upper blade (1) and lower blade (2) are made of transparent medical-grade plastic.
